(19) Europäisches Patentamt

European Patent Office

Office européen des brevets

(11) Veröffentlichungsnummer: **0 657 262 A2**

# (12) EUROPÄISCHE PATENTANMELDUNG

(21) Anmeldenummer: **94119121.5**

(22) Anmeldetag: **05.12.94**

(51) Int. Cl.6: **B29B 13/08**

---

(30) Priorität: **13.12.93 DE 4342391**

(43) Veröffentlichungstag der Anmeldung:
**14.06.95 Patentblatt 95/24**

(84) Benannte Vertragsstaaten:
**AT BE CH DE DK ES FR GB GR IT LI NL PT SE**

(71) Anmelder: **HOECHST AKTIENGESELLSCHAFT**

**D-65926 Frankfurt (DE)**

(72) Erfinder: **Rinno, Helmut, Dr.**
**Goethestrasse 38**
**D-65719 Hofheim (DE)**
Erfinder: **Hessel, Friedrich, Dr.**
**Raiffeisenstrasse 18**
**D-55124 Mainz (DE)**
Erfinder: **Alberti, Klaus, Dr.**
**Schöne Aussicht 20**
**D-65510 Idstein (DE)**

---

(54) **Verfahren zur Reduzierung der Keimzahl in wässrigen Kunstharzhaltigen Mehrphasensystemen mittels Mikrowellenstrahlen.**

(57) Verfahren zur Reduzierung der Keimzahl in mikrobiell kontaminierten wäßrigen Kunstharz-haltigen Mehrphasensystemen, wie Polymerdispersionen und polymere Bindemittel enthaltende Zubereitungen, durch dielektrische Erwärmung, vorzugsweise unmittelbar vor dem Abfüllen der Mehrphasensysteme.

EP 0 657 262 A2

Rank Xerox (UK) Business Services
(3.10/3.09/3.3.4)

Die vorliegende Erfindung betrifft ein Verfahren zur Reduzierung der Keimzahl in mikrobiell kontaminierten wäßrigen Kunstharz-haltigen Mehrphasensystemen, wie Polymerdispersionen und polymere Bindemittel enthaltende Zubereitungen, durch dielektrische Erwärmung.

Wäßrige Kunstharz-haltige Mehrphasensysteme, beispielsweise Polymerdispersionen und Dispersionsgebundene Systeme für den Klebstoff- oder Anstrichsektor sind durch mikrobiellen Befall und mikrobielle Zersetzung bedroht. Temperaturen um Raumtemperatur und pH-Werte im neutralen bis leicht alkalischen Bereich stellen gute Wachstumsbedingungen für Bakterien, Hefen und Pilze dar. Diese Mikroorganismen sind überall in der Umwelt anzutreffen. In Kunststoffdispersionen wurden verschiedene Mikroorganismen nachgewiesen (K.H. Wallhäuser, W. Fink, Die Konservierung von wäßrigen Kunststoff-Dispersionen, Farbe und Lack 91, 277 (1985)); Bakterien: Pseudomonaden, Enterobacteriaceae, Bacillusarten; Pilze: Aspergillus, Penicillium, Cephalosporium, Fusarium; Hefen: Saccharomyces. Ein ungehemmtes Wachstum solcher Mikroorganismen führt einerseits zu deutlich wahrnehmbaren äußerlichen Veränderungen, beispielsweise dem Auftreten von unangenehmem Geruch oder von sichtbarem Schimmelbewuchs, andererseits aber auch zu einer Veränderung wichtiger anwendungstechnischer Eigenschaften, beispielsweise der Viskosität oder des pH-Wertes.

Der angestrebte Wert für die Keimzahl liegt bei maximal 1000 Keimen pro ml, ein Wert, der den Richtlinien der Trinkwasserverordnung von 1986 (BGBl. I Nr. 22, 760, 1986) für Wasser von 18 - 22 °C aus Sammel- und Vorratsbehältern entspricht.

Zur Verhinderung des Befalls beziehungsweise zur Reduzierung der Gefahr eines Befalls mit Mikroorganismen werden verschiedene chemische oder physikalische Methoden beschrieben, beispielsweise in Wallhäuser, Praxis der Sterilisation, Desinfektion-Konservierung, 4. Aufl., Georg Thieme Verlag, Stuttgart, 1988. Diein der Praxis am häufigsten verwendete Methode zur Konservierung, beziehungsweise Sterilisation stellt der Zusatz von mikrobizid wirkenden Substanzen, wie Bakteriziden und Fungiziden dar. Die verwendeten chemischen Konservierungsmittel unterliegen strengen Zulassungsverfahren, es steht deshalb nur eine begrenzte Anzahl zur Verfügung. Sie dürfen aufgrund toxischer oder allergener Eigenschaften nur in geringen Mengen eingesetzt werden, können aber trotzdem bei empfindlichen Personen in Einzelfällen zu gesundheitlichen Störungen führen. Besonders wirksame Konservierungsmittel, beispielsweise Formaldehyd oder Formaldehyd-abspaltende Produkte, dürfen für eine Zubereitung gemäß BGA-Richtlinien in weiten Anwendungsbereichen überhaupt nicht verwendet werden. Ein weiteres Problem bei der Verwendung chemischer Konservierungsmittel ist die zunehmende Resistenz verschiedener Keime.

Wäßrige Kunstharz-haltige Mehrphasensysteme wie Polymerdispersionen, Dispersionsklebstoffe, Dispersionsfarben für Innen- und Außenanwendung, Grundierungen und Putze auf Dispersionsbasis, Betonhilfsmittel, Papierbeschichtungsmassen, Lederfinishes, Käsedeckmassen sowie Dispersionen von Polykondensationsprodukten, die in situ oder als Sekundärdispersion hergestellt werden, besitzen einen komplexen kolloidchemischen Aufbau, der diese Systeme aufgrund elektrostatischer, sterischer oder elektrosterischer Stabilisierungsmechanismen vor einer Phasentrennung schützt. Für die Erhaltung der gewünschten technischen Eigenschaften muß dieses System insbesondere hinsichtlich seiner stofflichen Zusammensetzung und seines Verteilungszustandes während der Herstellung, der Lagerung und des Transportes bis zur Applikation bestehen bleiben. Dies ist in der Regel nach einer massiven Vermehrung von Mikroorganismen nicht mehr der Fall. Es sind deshalb schon eine Reihe physikalischer und chemischer Behandlungsmethoden erprobt worden, um den Einsatz von Mikrobiziden zu vermeiden oder mengenmäßig zu begrenzen. Die Sterilisierung mittels bekannter physikalischer Methoden, beispielsweise Hitzebehandlung oder Behandlung mit $\gamma$-Strahlen führt zu einer Veränderung dieses komplexen kolloidchemischen Aufbaus. Die thermische Behandlung einer Polymerdispersion mittels indirektem Erhitzen führt erst bei hohen Temperaturen und Verweilzeiten (121 °C, 3 min) zu einer ausreichenden Keimreduzierung. Dabei kommt es allerdings schon nach kurzer Zeit durch Störung des kolloidchemischen Systems zu einer starken Belagbildung an den Wärmetauscherwänden, zur Koagulatbildung oder zur Änderung der Partikelgrößenverteilung. Die Belagbildung führt wiederum infolge eines erhöhten Wärmeübergangswiderstands zu weiterem Überheizen und damit zu vermehrten Ablagerungen. Diese Ablagerungen bestehen beispielsweie im Fall der Hitzebehandlung einer Dispersion aus koagulierten, nicht mehr redispergierbaren Polymerteilchen, im Fall der Hitzebehandlung einer Farbe aus koagulierten Polymerteilchen, die Pigmente und/oder Füllstoffe eingeschlossen haben. Die Verwendung von $\gamma$-Strahlen, beispielsweise aus einer $^{60}$Co-Quelle, führt in Anwesenheit von Sauerstoff und Wasser zu einer Bildung von Wasserstoffperoxid und von Hydroxyl-Radikalen, die unerwünschte Nebenreaktionen auslösen und eine Vernetzung des Polymers bewirken. Diese physikalischen Stabilisierungsmethoden kommen daher für eine technisch brauchbare Entkeimung von wäßrigen Kunstharz-haltigen Mehrphasensystemen nicht in Frage.

Zur Sterilisierung im Lebensmittelbereich oder für pharmazeutische Produkte ist die Verwendung von Mikrowellenstrahlung zur Erhitzung bekannt (K.H. Wallhäuser, Praxis der Sterilisation (4. Aufl.), Desinfektion - Konservierung, Georg Thieme Verlag, Stuttgart, 1988, S. 250). Auch Koch beschreibt die Verwendung der Mikrowellentechnik zum Sterilisieren von Lebensmitteln (K. Koch, Über den Einsatz der Mikrowellentechnik zum Haltbarmachen von Lebensmitteln, Lebensmitteltechnik, Band 1-2, S.39-43, 1989).

Im Lebensmittelsektor wird die Mikrowellenstrahlung ausschließlich verwendet, um die zu sterilisierenden Produkte auf eine bestimmte Temperatur zu erhitzen. Die für eine Inaktivierung/Abtötung der Mikroorganismen notwendige Expositionszeit muß beispielsweise in einem nachgeschalteten Warmhaltetunnel sichergestellt werden. Die in der Literatur angeführte notwendige Temperatur zum Sterilisieren von mindestens 121 °C entspricht dabei genau der Temperatur, die auch zum Sterilisieren mittels anderer thermischer Verfahren erforderlich ist. Somit sollte auch diese Methode für die komplexen Kunstharz-haltigen Mehrphasensysteme ungeeignet sein, da die erforderlichen hohen Temperaturen die bereits beschriebenen Veränderungen, insbesondere die Koagulation des komplexen Systems, auslösen.

Die Verwendung von Mikrowellenstrahlen zur Herstellung von Polymerdispersionen ist bereits aus US-A 3 432 413 bekannt. Die Reduzierung des Restmonomergehalts in Vinylhalogeniddispersionen mittels Mikrowellenstrahlung wird in US-A 4 117 220 und DE-A 26 12 572 beschrieben.

Aufgabe der vorliegenden Erfindung war also, ein Verfahren zur Reduzierung der Keimzahlen in wäßrigen Kunstharz-haltigen Mehrphasensystemen aufzufinden, das bereits bei moderaten Temperaturen wirkt und die aufgeführten Nachteile der bekannten Verfahren nicht aufweist.

Überraschenderweise zeigte sich, daß bei der dielektrischen Erwärmung eines wäßrigen Kunstharzhaltigen Mehrphasensystems durch Mikrowellenstrahlung die zur Keimreduzierung beziehungsweise Sterilisierung notwendigen Temperaturen deutlich niedriger liegen als bei konventionellem Energieeintrag, so daß eine das Produkt schädigende Temperaturerhöhung vermieden werden kann. Ebenfalls zeigte sich überraschend, daß durch Erhitzen von Dispersionen auf Temperaturen auch über 100 °C mit Hilfe der Mikrowellenstrahlung keine Störung des kolloidchemischen Zustandes wie Koagulatbildung oder Bildung von Wandbelag auftritt.

Erfindungsgegenstand ist daher ein Verfahren zur Reduzierung der Keimzahl in mikrobiell kontaminierten wäßrigen Kunstharz-haltigen Mehrphasensystemen durch dielektrische Erwärmung.

Die dielektrische Erwärmung der wäßrigen Mehrphasensysteme erfolgt vorzugsweise durch Mikrowelleneinstrahlung. Das erfindungsgemäße Verfahren kann mittels unterschiedlicher Mikrowellengeräte betrieben werden. Vorzugsweise wird eine Mikrowellenfrequenz von 0,04 bis 24 GHz, insbesondere von 0,4 bis 2,5 GHz genutzt. Ganz besonders bevorzugt sind die Mikrowellenfrequenzen von ca. 433 MHz, 915 MHz und 2,45 GHz.

Die Sterilisierung kann diskontiunuierlich (batchweise) durchgeführt werden. Vorzugsweise wird jedoch eine Mikrowellen-Apparatur zur kontinuierlichen Fahrweise benutzt, der gegebenenfalls eine Heißhaltestrekke nachgeschaltet werden kann. Die Arbeitsparameter, beispielsweise laminare oder turbulente Strömung, Einsatz von Mischern oder speziellen Pumpen sind von den jeweils eingesetzten Geräten abhängig, sind aber mit geringem Aufwand dahingehend zu optimieren, daß die wäßrigen Kunstharz-haltigen Mehrphasensysteme möglichst geringer Temperatur- und Scherbelastung ausgesetzt werden.

Die Sterilisationstemperaturen liegen unterhalb von 121 °C, vorzugsweise zwischen 50 und 100 °C, insbesondere zwischen 60 und 90 °C. Die Sterilisationszeiten hängen ab von der apparatebedingten Durchlaufmenge und der benötigten Temperatur und von der zur Verfügung stehenden Mikrowellenleistung. Die Sterilisation wird drucklos oder vorzugsweise unter erhöhtem Druck durchgeführt, um ein Aufschäumen oder eine Blasenbildung im Produkt zu verhindern. Der verwendete Druck zur Förderung im kontinuierlichen Prozeß und/oder zur Unterdrückung von Schaum- oder Blasenbildung beträgt vorzugsweise bis zu 10 bar, insbesondere 1 - 6 bar, über dem Normaldruck. Zur Vermeidung einer Blasenbildung, bei der es an der dem Gasraum ausgesetzten Oberfläche der Dispersion zu unerwünschter Hautbildung kommen kann, kann auch vorteilhaft von außen, beispielsweise durch die Produktförderpumpe zusammen mit einem Reduzierventil am Ende der Sterilisationsstrecke, aber auch durch ein Inertgas, ein erhöhter Systeminnendruck eingestellt werden. Die optimalen Parameter des erfindungsgemäßen Verfahrens müssen in den angegebenen Grenzen in Abhängigkeit von der jeweiligen Zusammensetzung des wäßrigen Kunstharz-haltigen Systems bestimmt werden. Insbesondere hängen Sterilisationstemperatur und -dauer von der Zusammensetzung und Beständigkeit des kolloidchemischen Systems und von den abzutötenden Keimen ab. Beispielsweise liegt die optimale Temperatur der Mikrowellensterilisation einer ®Mowilith DM 772-Polymerdispersion (Dispersion auf Basis von Acrylsäure/Methacrylsäureestern mit einem Feststoffgehalt von 46 Gew.-%, einer Viskosität von ca. 3000 mPa•s, einem pH-Wert ca. von 8,5 einer Dichte von ca. 1,06 g/cm$^3$) bei 70 °C bei einer Verweilzeit von 1 min in einer kontinuierlich betriebenen Mikrowellenanlage.

Vorzugsweise lassen sich Polymerdispersionen, die mittels Emulsionspolymerisation in Gegenwart von Radikalstartern hergestellt worden sind, beispielsweise Polyvinylchlorid, homo- und copolymere Polyvinylacetate, Polymere von Estern der Acrylsäure oder Methacrylsäure mit und ohne Schutzkolloid, Polystyrol, Copolymere von Styrol und Estern der Acryl/Methacrylsäure, Polybutadien, Copolymere von Styrol und Butadien oder wäßrige Dispersionen von Polykondensationsprodukten, wie Polyesterharzen, Polyurethanharzen, Alkydharzen, Epoxidharzen, und/oder Kunstharz-haltige Mehrphasensysteme, die diese Polymerdispersionen enthalten, beispielsweise Putz-, Klebstoff- oder Farbzubereitung mittels des erfindungsgemäßen Verfahrens entkeimen.

Ein besonderer Vorteil des erfindungsgemäßen Verfahrens liegt darin, daß wäßrige Kunstharz-haltige Mehrphasensysteme, die bei der Herstellung, bei der Lagerung, bei der Abfüllung, beim Transport oder bei anderen bei technischen Prozessen vielfach üblichen und unvermeidlichen Manipulationen während der Produktion bis zur endgültigen Verpackung der Einwirkung von Keimen ausgesetzt waren, durch eine Mikrowellenbehandlung sicher auf eine einheitlich sehr niedrige Keimzahl gebracht werden. Dies erlaubt es, die Produkte anschließend unter weitgehend sterilen Bedingungen gegebenenfalls ganz ohne Mikrobizidzusatz abzufüllen und bis zum Verbrauch in geschlossenen sterilen Gebinden zu lagern.

Eine solche Möglichkeit bestand bisher über einen längeren Zeitraum der Lagerung nicht, weil die Handhabung technischer Produkte wie Farben, Putze und dergleichen im gesamten Ablauf der Produktion und auch hinsichtlich aller verwendeten Rohstoffe nicht steril durchgeführt werden kann. Die Möglichkeit der Keimreduzierung in einem späten Stadium der Fabrikation von auf Polymerdispersionen basierenden Zubereitungen hat darüber hinaus den Vorteil, daß Keime, die von anderen Ausgangsprodukten als den Polymerdispersionen, beispielsweise von Pigmenten, Füllstoffen, Entschäumern, Dispergiermitteln und Verdickern in das System eingebracht werden, im fertigen Produkt ebenfalls abgetötet werden können.

Eine besondere Ausführungsform des erfindungsgemäßen Verfahrens liegt somit darin, daß Polymerdispersionen oder auf Polymerdispersionen basierende Zubereitungen erst unmittelbar vor dem Abfüllvorgang der Mikrowellenbehandlung unterworfen werden. Dabei kann entweder ganz auf den Zusatz von Mikrobiziden verzichtet werden, insbesondere wenn eine Abfüllung unter ausreichend sterilen Bedingungen in sterile Behältnisse erfolgt, oder die Menge an Mikrobizid wegen der geringen Keimzahl des Abfüllgutes auf weniger als 5O %, vorzugsweise weniger als 2O %, der sonst ohne Zwischenschaltung der Mikrowellenbehandlung notwendigen Mikrobizidmenge reduziert werden. Die erforderliche Mikrobizidmenge und die mögliche Reduktion wird durch Tests von Proben mit und ohne Mikrowellenbehandlung ermittelt. Beide Varianten des erfindungsgemäßen Verfahrens führen zu einer wesentlichen Verminderung der Exposition von Verbrauchern und Umwelt mit Mikrobiziden.

**Beispiele:**

Die in den folgenden Beispielen angegebenen Teile und Prozente beziehen sich auf das Gewicht, soweit nicht anders vermerkt.

Verwendete Produkte:

Dispersion A: ®Mowilith DM 772 Polymerdispersion auf Basis Acrylsäure-/ Methacrylsäureester, 46 % Feststoffgehalt, Viskosität 3000 mPa•s, pH-Wert 8,5, Dichte 1,06 g/cm$^3$

Dispersion B: ®Mowilith DM 2KL Dispersion auf Basis Vinylacetat/Maleinsäureester mit Schutzkolloid, 45 % Feststoffgehalt, Viskosität 9000 mPa•s, pH-Wert 5, Dichte 1,15 g/cm$^3$

Farbe:

Dispersionsfarbe für außen und innen, gemäß DIN 53778, enthaltend 100 Teile Mowilith DM 772, 10 Teile Wasser, 0,4 Teile Ammoniak konz., 5,5 Teile 10%ige wäßrige Lösung von ®Calgon 135 (Hersteller Benkiser, Ladenburg), 0,4 Teile ®Genapol PN 30 (Hersteller Hoechst AG, Frankfurt), 0,5 Teile Entschäumer ®Agitan 295 (Hersteller Münzing, Heilbronn), 1,8 Teile 5 %ige wäßrige Lösung von ®Borchigel L75 (Hersteller Fa. Borchers AG Düsseldorf), 36,4 Teile Titandioxid ®Kronos CL 300 (Hersteller Fa. Kronos Titan, Leverkusen), 18,2 Teile ®Omya Hydrocarb 90 (Hersteller Fa. Omya, Köln), 6,5 Teile 1,2-Propylenglykol, 1,8 Teile Butyldiglykolacetat. Der Festkörpergehalt beträgt ca. 55 %, die Pigmentvolumenkonzentration (PVK) liegt bei 28 %, die Dichte bei 1,3 g/cm$^3$.

Keimzahlbestimmung, KBE (Kolonie-bildende Einheiten):

Die Probe wird unter sterilen Bedingungen verdünnt (Verdünnungsfaktor 1: 100) und auf zwei verschiedene Fertignährböden (CSA-Agar für Bakterien, SDA-Agar für Hefe-Pilze, Hersteller Fa. Merck, Darmstadt) aufgetragen und für 5 Tage bei 30 °C in einem temperierten Brutschrank gelagert. Die Auswertung erfolgt

EP 0 657 262 A2

durch Auszählen der Kolonien, Nachweisgrenze < 100 KBE/ml.

1. Thermische Behandlung der Dispersionen A und B:

Die Dispersionen A und B werden durch thermische Behandlung in einem Autoklaven für kurze Zeit auf verschiedene Temperaturen erhitzt, anschließend wird die Keimzahl bestimmt. Bei Temperaturen über 100 °C werden die Dispersionen irreversibel geschädigt. Die Ergebnisse sind in Tabelle 1 zusammengestellt.

Tab. 1

| Keimzahlen von wäßrigen Kunstharz-haltigen Mehrphasensystemen vor/nach thermischer Behandlung | | | | | |
|---|---|---|---|---|---|
| | Behandlung | | CSA | SDA | Bemerkung |
| | Temp. [°C] | Zeit [min] | [KBE/ml] | | |
| Dispersion A | unbehandelt | | $1 \cdot 10^6$ | $1 \cdot 10^5$ | |
| Dispersion A | 121 | 3 | < 100 | < 100 | Koagulat, Wandbelag |
| Dispersion A | 121 | 1 | 800 | 500 | Koagulat, Wandbelag |
| Dispersion A | 100 | 1 | 1500 | 1000 | Wandbelag |
| Dispersion A | 80 | 5 | $1 \cdot 10^4$ | $1 \cdot 10^4$ | |
| Dispersion B | unbehandelt | | $1 \cdot 10^5$ | $1 \cdot 10^5$ | |
| Dispersion B | 121 | 3 | < 100 | < 100 | Koagulat, Wandbelag |
| Dispersion B | 121 | 1 | 500 | 900 | Koagulat, Wandbelag |
| Dispersion B | 100 | 1 | 2000 | 2500 | Wandbelag |
| Dispersion B | 80 | 5 | $1 \cdot 10^4$ | $1 \cdot 10^4$ | |

2. Diskontinuierliche (batchweise) Sterilisierung mit Mikrowellenstrahlung

Verschiedene wäßrige Kunstharz-haltige Mehrphasensystemen, die eine hohe Ausgangskeimzahl aufwiesen, werden batchweise in einem Mikrowellengerät (MLS 1200 Fa. Büchi Laboratoriumstechnik, Göppingen) behandelt. 10 g der jeweiligen Proben werden in einem ®Teflonbehälter für 10 min (Tab. 2), beziehungsweise für 2 min (Tab. 3) mit Mikrowellenstrahlen (2,45 GHz) behandelt. Die sich einstellende Temperatur ist dabei ein Maß für die Intensität der Mikrowellenbestrahlung.

Die Probenbehälter weisen nach der Versuchsdurchführung weder Wandbelag noch Koagulat auf, die kolloidchemische Zustand der Dispersionen wird durch die indirekte Erhitzung nicht verändert. Die Proben werden unter sterilen Bedingungen abgefüllt und auf noch vorhandene Keimgehalte überprüft.

5

Tab.2: Keimzahlen von wäßrigen Kunstharz-haltigen Mehrphasensystemen bei Mikrowellenbehandlung (10 min, pulsierende MW-Strahlung so eingestellt, daß die unten angegebenen Temperaturen erreicht werden)

| Produkt | unbehandelt | | mit Mikrowellen behandelt | | | | | | | |
|---|---|---|---|---|---|---|---|---|---|---|
| | | | 80 °C | | 90 °C | | 100 °C | | 120 °C | |
| | CSA | SDA | CSA | SDA | CSA | SDA | CSA | SDA | CSA | SDA |
| | [KBE/ml] | | [KBE/ml] | | [KBE/ml] | | [KBE/ml] | | [KBE/ml] | |
| Dispersion A | $1 \cdot 10^4$ | $1 \cdot 10^6$ | <100 | <100 | <100 | <100 | <100 | <100 | <100 | <100 |
| Dispersion B | $1 \cdot 10^4$ | $1 \cdot 10^4$ | <100 | <100 | <100 | <100 | <100 | <100 | <100 | <100 |
| Farbe | $1 \cdot 10^5$ | $1 \cdot 10^5$ | <100 | <100 | <100 | <100 | <100 | <100 | <100 | <100 |

Tab.3: Keimzahlen von wäßrigen Kunstharz-haltigen Mehrphasensystemen bei Mikrowellenbehandlung (2 min, pulsierende MW-Strahlung so eingestellt, daß die unten angegebenen Temperaturen erreicht werden)

| Produkt | unbehandelt | | mit Mikrowellen behandelt | | | | | | | |
|---|---|---|---|---|---|---|---|---|---|---|
| | | | 60 °C | | 70 °C | | 80 °C | | 90 °C | |
| | CSA | SDA | CSA | SDA | CSA | SDA | CSA | SDA | CSA | SDA |
| | [KBE/ml] | | [KBE/ml] | | [KBE/ml] | | [KBE/ml] | | [KBE/ml] | |
| Dispersion A | $1 \cdot 10^5$ | $1 \cdot 10^4$ | 200 | <100 | 600 | <100 | 400 | <100 | 300 | <100 |
| Dispersion B | $1 \cdot 10^5$ | $1 \cdot 10^5$ | 300 | <100 | 600 | <100 | 500 | 300 | <100 | <100 |

2. Kontinuierliche Sterilisierung

Die wäßrigen Kunstharz-haltigen Mehrphasensysteme werden aus einem Vorratsgefäß mit einer selbstansaugenden Mohnopumpe mit einer geringen Scherbeanspruchung gegen einen Überdruck von 1 bar durch die Mikrowellen-Versuchsanlage gefördert (Anlage wie oben beschrieben, mit zwei Produktdurchführungsmöglichkeitenrechts und links). Die Verweilzeit in der Mikrowellenaufheizstrecke beträgt 10 bis 180 sec. An die Aufheizstrecke schließt sich eine Heißhaltestrecke mit unterschiedlich langen Verweilzeiten an. Die Sterilisationen werden bei Verweilzeiten von 1, 3 und 5 min im Temperaturbereich zwischen 60 und 80 °C durchgeführt. Die Probetemperatur wird direkt nach der Aufheizstrecke sowie am Ende der Heißhaltestrecke gemessen. Die Temperatur wird über den Mikrowelleneintrag geregelt und ist damit proportional zur Intensität der Mikrowellenbestrahlung.

Die Ergebnisse der Versuche mit verschiedenen Produkten sind in Tabelle 4 zusammengestellt. Die Keimzahlbestimmung erfolgt wie oben beschrieben.

Tab. 4

| Keimzahlen von wäßrigen Kunstharz-haltigen Mehrphasensystemen im kontinuierlichen Mikrowellenversuch mit/ohne Heißhaltestrecke (pulsierende MW-Strahlung so eingestellt, daß die unten angegebenen Temperaturen erreicht werden, 1 min Mikrowellenbehandlung (MW), 3 l/h Durchsatz, 1,8 bar) | | | | | | |
|---|---|---|---|---|---|---|
| Temp [°C] | MW [min] | MW + Heißhaltezeit [min] | Dispersion A | | Dispersion B | |
| | | | CSA | SDA | CSA | SDA |
| | | | [KBE/ml] | | [KBE/ml] | |
| unbehandelt | | | $1 \cdot 10^4$ | $1 \cdot 10^4$ | 3300 | $1 \cdot 10^4$ |
| 60 | 1 | -- | 3200 | 1900 | 100 | < 100 |
| 60 | 1 | 1 | 3900 | 200 | < 100 | < 100 |
| 60 | 1 | 3 | 3200 | 200 | < 100 | < 100 |
| 60 | 1 | 5 | 1300 | 200 | < 100 | < 100 |
| 70 | 1 | -- | < 100 | < 100 | < 100 | < 100 |
| 70 | 1 | 1 | < 100 | < 100 | 300 | < 100 |
| 70 | 1 | 3 | < 100 | < 100 | < 100 | < 100 |
| 70 | 1 | 5 | 100 | < 100 | < 100 | < 100 |
| 80 | 1 | -- | < 100 | < 100 | < 100 | < 100 |
| 80 | 1 | 1 | < 100 | < 100 | < 100 | 200 |
| 80 | 1 | 3 | < 100 | < 100 | 100 | < 100 |
| 80 | 1 | 5 | < 100 | < 100 | 100 | 100 |

Die Mikrowellenbehandlung wird unter einem erhöhten Druck von 9 bar in der beschriebenen kontinuierlich betriebenen Anlage durchgeführt. Die Ergebnisse der Versuche sind in Tabelle 5 zusammengestellt.

Tab. 5

| Temp [°C] | MW [min] | MW + Heißhaltezeit [min] | Dispersion A | | Dispersion B | |
|---|---|---|---|---|---|---|
| Keimzahlen von wäßrigen Kunstharz-haltigen Mehrphasensystemen im kontinuierlichen Mikrowellenversuch mit /ohne Heißhaltestrecke (pulsierende MW-Strahlung so eingestellt, daß die unten angegebenen Temperaturen erreicht werden, 1 min Mikrowellenbehandlung (MW), 3 l/h Durchsatz, 9 bar) | | | | | | |
| | | | CSA | SDA | CSA | SDA |
| | | | [KBE/ml] | | [KBE/ml] | |
| unbehandelt | | | $1 \cdot 10^4$ | $1 \cdot 10^4$ | 3300 | $1 \cdot 10^4$ |
| 70 | 1 | -- | < 100 | < 100 | < 100 | < 100 |
| 70 | 1 | 1 | < 100 | < 100 | < 100 | < 100 |
| 70 | 1 | 3 | < 100 | < 100 | < 100 | < 100 |
| 70 | 1 | 5 | < 100 | < 100 | < 100 | < 100 |

In einem weiteren Experiment wird eine Farbe, die von Mikroorganismen befallen ist, in der beschriebenen kontinuierlich betriebenen Mikrowellenanlage mit dielektischer Strahlung behandelt. Die Ergebnisse sind in Tabelle 6 zusammengestellt.

Tab. 6

| Temp. [°C] | MW [min] | MW + Heißhaltezeit [min] | Farbe | |
|---|---|---|---|---|
| | | | CSA | SDA |
| | | | [KBE/ml] | |
| unbehandelt | | | $1 \cdot 10^5$ | $1 \cdot 10^4$ |
| 50 | 1 | - | $1 \cdot 10^5$ | $1 \cdot 10^4$ |
| 50 | 1 | 3 | $1 \cdot 10^5$ | $1 \cdot 10^4$ |
| 60 | 1 | - | 900 | 800 |
| 60 | 1 | 3 | 800 | 900 |
| 70 | 1 | - | 200 | < 100 |
| 70 | 1 | 3 | 300 | < 100 |
| 80 | 1 | - | < 100 | < 100 |
| 80 | 1 | 3 | < 100 | < 100 |

Keimzahlen eines wäßrigen Kunstharz-haltigen Mehrphasensystems in Form einer Farbe im kontinuierlichen Mikrowellenversuch mit /ohne Heißhaltestrecke (pulsierende MW-Strahlung so eingestellt, daß die unten angegebenen Temperaturen erreicht werden, 1 min Mikrowellenbehandlung (MW), 3 l/h Durchsatz, 1,8 bar)

Die Ergebnisse der Untersuchungen zeigen, daß durch die Mikrowellenbehandlung von verschiedenen wäßrigen Kunstharz-haltigen Mehrphasensystemen schon bei Temperaturen oberhalb von 60 °C und deutlich unterhalb von 100 °C eine Reduktion der Keimzahlen von $10^6$ KBE/ml bis auf einige 100 Keime oder sogar bis unter die Nachweisgrenze möglich ist. Die Ergebnisse zeigen ebenfalls, daß die Abtötung der Mikroorganismen in erster Linie von der Intensität der Mikrowellenstrahlung abhängt. Die Länge der Heißhaltestrecke spielt offensichtlich nur eine geringe Rolle. Auch die Sterilisierung mit Hilfe der Mikowellenstrahlung und höheren Drücken führt ebenfalls zu einer Keimreduzierung unter die Nachweisgrenze, ohne daß die Produkteigenschaften negativ beeinflußt werden.

In dem kontinuierlich betriebenen Mikrowellenbestrahlungsversuch wird keine Belagbildung an den Innenwänden der Rohrleitung der Versuchsanlage festgestellt. Weder bei der kontinuierlichen noch bei der diskontinuierlichen Mikrowellenbestrahlung werden die Produkte in ihren produktspezifischen Eigenschaften geschädigt.

**Patentansprüche**

1.  Verfahren zur Reduzierung der Keimzahl in mikrobiell kontaminierten wäßrigen Kunstharz-haltigen Mehrphasensystemen durch dielektrische Erwärmung.

2.  Verfahren nach Anspruch 1, dadurch gekennzeichnet, daß die dielektrische Erwärmung mittels Mikrowellenstrahlen erfolgt.

3.  Verfahren nach Anspruch 2, dadurch gekennzeichnet, daß die Mikrowellenfrequenz 0,04 bis 24 GHz beträgt.

4.  Verfahren nach Anspruch 2, dadurch gekennzeichnet, daß das kunstharzhaltige Mehrphasensystem den Mikrowellenstrahlen gemäß Anspruch 3 ausgesetzten Raum kontinuierlich durchströmt.

5.  Verfahren nach Anspruch 1, dadurch gekennzeichnet, daß die Sterilisationstemperatur unterhalb von 121 °C liegt.

6.  Verfahren nach Anspruch 5, dadurch gekennzeichnet, daß die Sterilisationstemperatur zwischen 50 und 100 °C beträgt.

7.  Verfahren nach Anspruch 1, dadurch gekennzeichnet, daß die Sterilisation unter einem sich einstellenden Druck von bis zu 10 bar durchgeführt wird.

8.  Verfahren nach Anspruch 1, dadurch gekennzeichnet, daß als wäßriges Kunstharz-haltiges Mehrphasensystem eine Polymerdispersion verwendet wird.

9.  Verfahren nach Anspruch 8, dadurch gekennzeichnet, daß als Polymerdispersion Acrylat- oder Vinylacetatgruppen-haltige Emulsionspolymerisate verwendet werden.

10.  Verfahren nach Anspruch 1, dadurch gekennzeichnet, daß als wäßriges Kunstharz-haltiges Mehrphasensystem eine Zubereitung, enthaltend eine Polymerdispersion, verwendet wird.

11.  Verfahren nach Anspruch 1, dadurch gekennzeichnet, daß die Mikrowellenbehandlung unmittelbar vor dem Abfüllen der hergestellten Polymerdispersionen oder auf Polymerdispersionen basierenden Folgeprodukte erfolgt.

12.  Verfahren nach Anspruch 11, dadurch gekennzeichnet, daß den hergestellten Polymerdispersionen oder auf Polymerdispersionen basierenden Folgeprodukten zusätzlich weniger als 50 % der ohne Mikrowellenbehandlung notwendigen Menge eines Mikrobizids zugesetzt werden.